# EUROPEAN PATENT SPECIFICATION

(11) **EP 2 625 271 B1**
(45) Date of publication and mention of the grant of the patent: **31.05.2017**
(21) Application number: 11770551.7
(22) Date of filing: 04.10.2011
(51) Int. Cl.: C12N 15/10, C12Q 1/68

(54) **METHOD FOR EXTRACTING DNA FROM NEMATODES AND PLANT CELLS**
VERFAHREN ZUR EXTRAKTION VON DNA AUS NEMATODEN UND PFLANZENZELLEN
MÉTHODE D'EXTRACTION D'ADN DE CELLULES VÉGÉTALES ET DE NÉMATODES

(30) Priority: 04.10.2010 EP 10186370
(43) Date of publication of application: 14.08.2013
(73) Proprietor: Tree of Knowledge Patents B.V., 6709 PA Wageningen (NL); Wageningen Universiteit, 6708 PB Wageningen (NL)
(72) Inventor: HELDER, Johannes, 6707 AP Wageningen (NL)
(74) Representative: V.O.
(86) International application number: PCT/NL2011/050670
(87) International publication number: WO 2012/047099

(56) References cited:
- US-A- 5 925 565
- US-A1- 2006 035 324
- PASTRANA DIANA V ET AL: "Filarial nematode parasites secrete a homologue of the human cytokine macrophage migration inhibitory factor", INFECTION AND IMMUNITY, vol. 66, no. 12, December 1998 (1998-12), pages 5955-5963, XP002609983, ISSN: 0019-9567
- HELEN F MCGARRY ET AL.: "Evidence against Wolbachia symbiosis in Loa loa", FILARIA JOURNAL, vol. 2, no. 1, 2 May 2003 (2003-05-02), XP002609984,
- OLSSON ET AL: "Molecular and anatomical evidence for a three-way association between Pinus sylvestris and the ectomycorrhizal fungi Suillus bovinus and Gomphidius roseus", MYCOLOGICAL RESEARCH, ELSEVIER, GB, vol. 104, no. 11, 1 November 2000 (2000-11-01), pages 1372-1378, XP022454182, ISSN: 0953-7562, DOI: DOI:10.1017/S0953756200002823

## Description

### FIELD OF THE INVENTION

The present invention is in the field of sample preparation for molecular analysis, in particular to methods for extracting DNA from lysis-resistant organisms, including nematodes and plant cells.

### BACKGROUND OF THE INVENTION

Nematodes (roundworms) are non-segmented invertebrate animals that cover an entire phylum (Phylum Nematoda) which is estimated to consist of more than 5 million different species. Of all nematodes, the plant-parasitic nematodes that cause root galls, rots and lesions and that can severely retard root growth by feeding on plant roots are the best known. Yet they form a minority within the phylum. The majority of nematodes are free living meiofauna of terrestrial soils and freshwater and marine sediments. The number of individuals which can be found in a soil sample can be between 2 and 20 million per square meter with a sample of approximately 100 ml harboring between 30 and 60 different species.

Native populations of nematodes in the soil offer the possibility of determining the health of the soil ecosystem. Apart from their presence in high numbers and to great depth (up to 10 meters) this is due to the fact that nematodes as a group are very diverse. Moreover, the duration of the life cycle of nematodes is very diverse.

Nematodes have been classified and are accordingly still identified by virtue of subtle morphological features. However, there are very few experts who are competent in the morphological identification of nematodes and are capable of completely analyzing a nematode population in a particular soil sample on the basis of solely morphological features. Here, it should also be noted that, in a large number of cases, nematodes in larval stages (that is, non-mature specimens) cannot be identified, not even by taxonomic experts.

Apart from the lack of experts in this field, the analysis of one single sample and the identification therein of approximately 150 (mature) individuals, takes about 3-4 hours on average.

It is known that certain drawbacks of morphological identification methods can be obviated by use of molecular identification methods. For instance, sequence data of the so-called small subunit (SSU) gene located in the ribosomal RNA cistron or mitochondrial sequence data can be used to determine the evolutionary relationship and/or the genetic diversity of nematodes. Molecular identification methods based on (RT-)PCR methods require the availability of isolated, or at least enzyme-accessible DNA. However, the physical and biochemical characteristics of the nematode exoskeleton have hampered the development of a versatile, affordable & high throughput protocol for the quantitative isolation of DNA from nematodes.

Nematodes lack a true skeleton and a circular muscle system. Nematodes move by the coordinated contraction of longitudinal muscles (agonists) positioned just inside the hypodermis. Nematodes typically lack antagonistic muscles, and the nematode body maintains its shape by means of a hydroskeleton; the interior of the nematode is fluid-filled and under high pressure (functionally replacing antagonistic muscles). The outer body layer - the cuticle - is a collagen-rich, multilayered structure and as such it imparts the strength needed to compensate for the high internal pressure. Moreover, the cuticle functions as an exoskeleton, defining the shape of the animal, protecting the nematode against sharp soil particles, and it provides the nematode resistance to desiccation and invasion by bacteria and fungi, and resistance to host defense responses in many parasitic species. As such this outer layer is physically strong, and recalcitrant towards chemical and / or biological degradation.

There is presently a need for a quick, versatile, sensitive, affordable, and easy to perform DNA extraction method that requires minimal sample handling for nematodes and in particularly for nematode communities that is capable of making DNA of individual nematode cells quantitatively available for PCR such that nematode numbers can be quantified in soil. Moreover it is desirable that this DNA extraction method is applicable to a wide variety of nematodes and is essentially unbiased with respect to species identity, meaning that DNA is extracted with similar efficiencies for different species. The prior art methods are not capable of providing such a quantitative DNA extraction procedure that allows quantitative nucleic acid amplification of the material thus attained.

### SUMMARY OF THE INVENTION

The present inventors have now established a method for extracting nucleic acids from nematodes, preferably terrestrial and freshwater nematodes, that solves the problems of the prior art methods.

Concomitantly, the present inventors have found that this same lysis solution is also very suitable for the extraction of nucleic acids from plant cells.

An important advantage of the lysis composition of the present invention is that the nucleic acids thus isolated can be used directly (hence, without any physical and / or mechanical pre-treatment), usually with dilution of the lysate for up to 100x or more, in a PCR reaction for amplifying and further analysing said nucleic acids. The lysis composition is not only very suitable for the recovery and amplification of DNA, but also for the recovery and subsequent quantitative amplification of RNA.

In particular the invention provides in a first aspect a composition for lysis of nematodes as defined in claim 1, consisting of an aqueous buffer containing 0.01-0.5 wt % of a reducing agent, 0.01-0.5 wt% of the endopeptidase Proteinase K, 0-0.5 wt% of a chelating agent, and 0.001-0.1 wt% of an anionic surfactant, wherein said composition, upon lysis of the nematodes therein, provides a nucleic acid template solution for use in a subsequent amplification reaction without further purification.

A highly preferred composition comprises in an aqueous buffer 0.5 wt % of a reducing agent, 0.04 wt% of the endopeptidase, 0-0.15 wt% of chelating agent, and 0.025 wt% of anionic surfactant.

In a further preferred embodiment, the composition comprises 0.025 wt% SDS (0.9 mM); 0.5% (v/v) 2-mercaptoethanol (70 mM) and 400 mg/L proteinase K in an aqueous buffer, optionally further comprising 1-5 mM EDTA (0.03 - 0.15 wt%).

Still further preferred is a composition comprising in an aqueous buffer 0.5 wt% of 2-mercaptoethanol, 0.04 wt% of proteinase K, 0-0.03 wt% of EDTA, and 0.025 wt% of SDS, and wherein said aqueous buffer comprises 0.6 wt% of NaCl and 1.5 wt% of Tris-HCl pH 8.0.

In another aspect, the present invention provides a method of lysing a nematode or plant cell, said method comprising exposing the nematode or plant cell to a composition of the invention as described above for a sufficient amount of time for lysis to occur. The result of said lysis is that the genomic material from said nematode is liberated from said nematode without physical and / or mechanical disruption and without a requirement for additional purification steps.

In another aspect, the present invention provides a method for isolating nucleic acids from a nematode or plant cell, said method comprising exposing the nematode or plant cell to a composition of the invention as described above for a sufficient amount of time for lysis to occur in order to provide a nematode or plant cell lysate and recovering nucleic acids from said lysate.

In a preferred embodiment of a method for isolating nucleic acids according to the present invention, said exposure is maintained for between 0.5 and 4 hours at a temperature of between 35 and 70 °C, preferably 2 hours at 65 °C.

In another aspect, the present invention provides a method of amplifying a nucleic acid of interest from a nematode or plant cell, said method comprising providing a nucleic acid by a method for isolating nucleic acids from a nematode or plant cell as described above and subjecting the nucleic acid thus provided to conditions that result in amplification of the nucleic acid of interest.

In a preferred embodiment of a method of amplifying a nucleic acid of interest according to the present invention, said wherein subjecting comprises performing a PCR amplification of the nucleic acid of interest.

In a preferred embodiment of a method of amplifying a nucleic acid of interest according to the present invention, said method further comprises, prior to amplifying, the step of reverse transcribing the RNA in said nucleic acid into cDNA.

In another aspect, the present invention provides a method for producing a composition for lysis of nematodes or plant cells as described above, said method comprising mixing the said reducing agent, the endopeptidase Proteinase K, chelating agent, and anionic surfactant in the indicated amounts in an aqueous buffer to form said composition.

Also described herein is a kit-of-parts comprising an instruction (in written, printed or digital form) for performing a method of the present invention as described above, and optionally further comprising a container comprising at least one selected from a reducing agent, an endopeptidase, a chelating agent, an anionic surfactant, a nucleic acid and an aqueous buffer.

In a preferred embodiment of such a description, said kit further comprising, in packaged combination, a composition for lysis of nematodes or plant cells as described above in one or more containers. The said packaged combination indicates that the individual components of the composition may be provided in individual containers but are packaged in combination (e.g. in a box). This facilitates the production of the composition of the invention by the end user through mixing (a part of) the content of the individual containers in a desired ratio so as to obtain the effective lysis solution. Effective meaning that the solution provides for the effect of lysing the nematodes as indicated herein. In such a kit, the (four) individual components are preferably provided in one or more separate containers, although combination of some of the components (for instance two or three) is possible as long as the stability of the individual components is not negatively affected. Thus, a composition of the invention may be provided in a kit in a single container, and more than one container in a kit may contain the composition of the invention. Alternatively, the components of the invention may be provided in one or more separate containers, together with an instruction for mixing the individual components in the desired ratios to obtain the composition of the invention.

In a preferred embodiment of a kit as described herein, the nucleic acid comprises at least one primer for amplification of a nucleic acid sequence of interest.

In still another preferred embodiment of a kit as described herein, the kit further comprises one or more containers containing some or all of the reagents necessary for amplification of a nucleic acid sequence of interest.

In yet another preferred embodiment of a kit as described herein, the kit further comprises one or more containers containing some or all of the reagents necessary for performing a PCR reaction.

### DESCRIPTION OF THE DRAWINGS

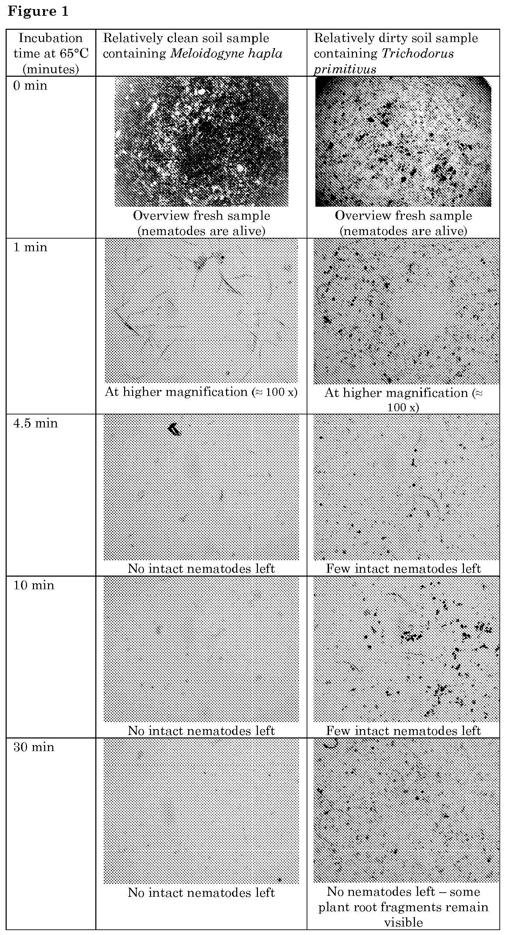

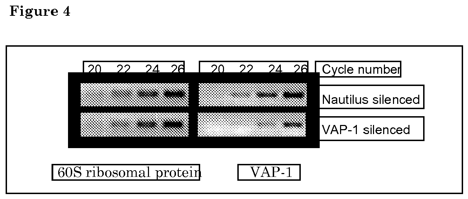

Figure 1. Extraction of DNA from nematodes. Time series showing the impact of the lysis solution according to the invention on two nematode species, *viz*. *Meloidogyne hapla* and *Trichodorus primitivus* (magnification ≈ 100 x). It is noted that, as compared to *M. hapla,* lysis of *T. primitivus* requires longer exposure to the lysis solution.

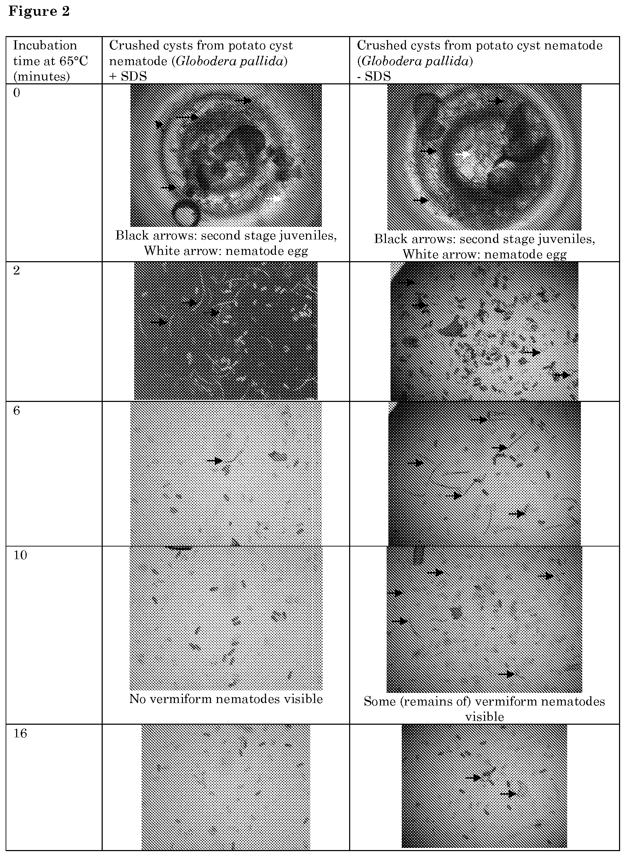

Figure 2. Extraction of DNA from nematodes; effect of an anionic detergent. Time series showing the impact of the lysis solution as described in this document and one variant namely the lysis solution without SDS on pre-parasitic second stage juveniles of the potato cyst nematode *Globodera pallida* (magnification ≈ 100 x). It is noted that the presence of SDS increases the efficiency of the lysis solution.

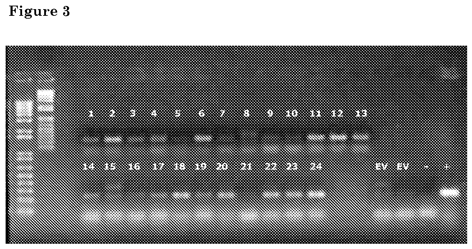

Figure 3. Extraction of DNA from plant material.
   Ethidium-stained agarose gel showing mIL-10-KDELpc-derived PCR products amplified from leaf material (samples of ≈ 5 mg fresh leaf material each) of 24 independent stable transformants of *Nicotiana tabacum* incubated in lysis solution as described in this document. EV = empty vector (negative control plants); "-" = water control; "+" positive control (= construct in vector). Two lanes at the extreme left - DNA size ladders
Figure 4. Extraction of RNA from (plant parasitic) nematodes Ethidium-stained agarose gel showing RT-PCR products from dsRNA-treated pre-parasitic juveniles of the potato cyst nematode *Globodera rostochiensis*. "60S ribosomal protein": RT-PCR product from mRNA encoding a fragment of the 60S ribosomal protein (fragment; internal control). Treated juveniles were exposed to lysis solution as described in this document for 30 min. "VAP-1" RT-PCR product from mRNA encoding a part of VAP-1, a potato cyst nematode gene. "Nautilus silenced": check for non specific effects of dsRNA (potato cyst nematodes do not harbour the corresponding gene)

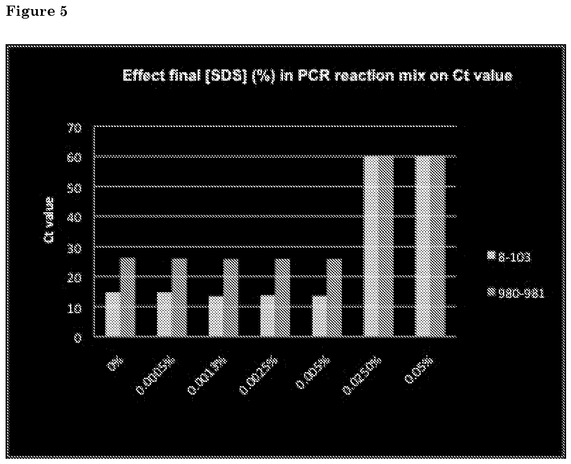

Figure 5. Effect of SDS concentration in lysis buffer on the lysis and PCR-based amplification of pre-parasitic juveniles of the potato cyst nematode G. *rostochiensis*. As compared to other nematode species and life stages, this particular nematode-life stage combination is notorious for being extremely hard to lyse. Primer combination 8 - 103 will amplify a fragment of SSU rDNA of *G. rostochaensis*, whereas primer combination 980-981 is used as an internal control (it will amplify a standard quantity of control DNA). On the y-axis the Ct values are given (output of Real Time - PCR). The most optimal lysis buffer composition is characterized by the lowest Ct value.

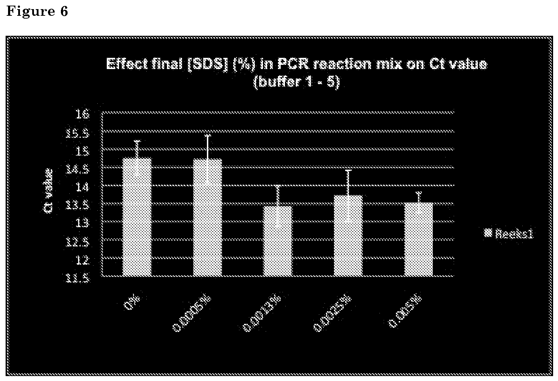

Figure 6. In order to better visualize the Ct differences, the two highest SDS concentration (0.0250% and 0.05%) - concentrations that resulted in a virtually full blockage of the PCR reactions both for target organism and internal control- were removed from Figure 5. Moreover, this figure shows the Ct values produced by PCR primer combination 8 - 103 that uses *G. rostochiensis* DNA as template.

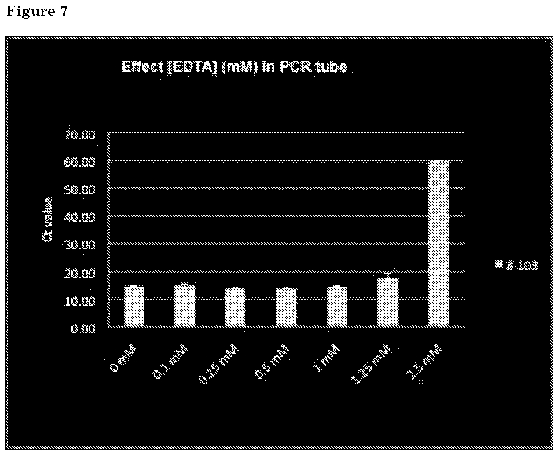

Figure 7. Effect of EDTA concentration in lysis buffer on the lysis and PCR-based amplification of pre-parasitic juveniles of the potato cyst nematode *G. rostochiensis*. As compared to other nematode species and life stages, this particular nematode-life stage combination is notorious for being extremely hard to lyse. Primer combination 8 - 103 will amplify a fragment of SSU rDNA of *G. rostochiensis*. On the y-axis the Ct values are given (output of Real Time - PCR). The most optimal lysis buffer composition is characterized by the lowest Ct value.

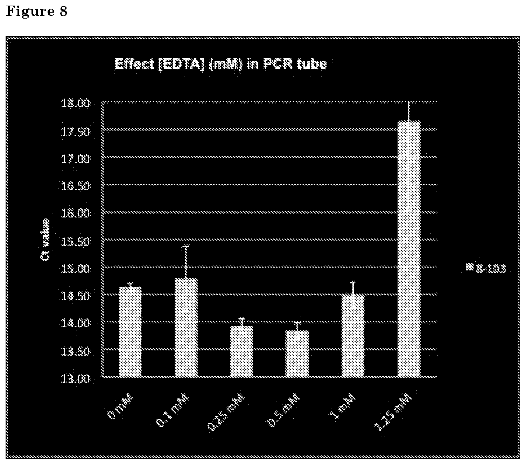

Figure 8. In order to better visualize the Ct differences, the highest EDTA concentration (2.5 mM EDTA) - a concentration that resulted in a virtually full blockage of the PCR reactions were removed from Figure 7. The most optimal lysis buffer composition is characterized by the lowest Ct value.

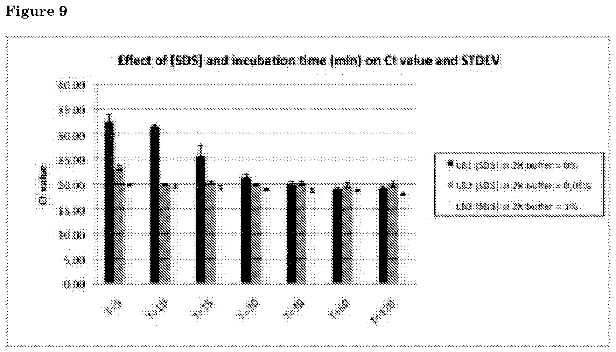

Figure 9. The effect of lysis time, incubation at 65°C while shaking the Eppendorf vial with target organisms and lysis buffer at 750 rpm in a Thermomixer (Eppendorf, Germany), on lysis efficiency as quantified by Ct value (on the y-axis).

### DETAILED DESCRIPTION OF THE INVENTION

Weight percentages (wt %) as used throughout this specification refer to weight/volume (w/v) percentages when reference is made to solutions. w/w % are intended in all other cases. Weight percentages refer to the percentage of the ingredient based on the entire weight of the composition.

Compositions for lysis of prokaryotic and eukaryotic cells are generally known in the art of nucleic acid isolation. Typically, such compositions provide an environment for preservation of the nucleic acids liberated from the cells, as well as an environment for the separation of the nucleic acids from other cell components. When chemical lysis is used, such compositions include organic solvents or detergents that dissolve or disrupt cellular membranes or cell walls in addition to compounds that allow nucleic acids of interest to be separated from other components. Chemical lysis compositions are therefore inherently more complex than mechanical lysis compositions.

Among the mechanical methods known for lysing cells, mechanical disruption (e.g., mechanical blender, glass beads, grinding of frozen cells), liquid homogenization (e.g., French pressure cell, Dounce homogenizer), high frequency sound waves (e.g., sonication), and freeze/thaw cycles (e.g., dry ice baths, liquid nitrogen baths) are the most commonly used.

Common chemical methods, which are used alone or in combination with mechanical methods, include use of hypotonic buffers and use of cell wall degrading enzymes, such as lysozyme. Depending on the ultimate use for the lysed cells, other macromolecule-degrading substances are often included in the lysis buffers. For example, if cells are being lysed for capture of nucleic acids, proteases can be added to degrade proteins that might interfere with isolation of the nucleic acids.

Protocols for lysis of cells and isolation of nucleic acids are also known in the art. Many involve exposing the cells to organic solvents, such as phenol and chloroform, and later removing traces of these organics to enable enzymatic manipulation of the nucleic acids. Others involve exposing cells to detergents and other substances that dissolve membranes and/or cause osmotic pressure to lyse cells. Among the many detergents used is sodium dodecyl sulfate (SDS). The combination of SDS and sodium hydroxide has been known for years to be suitable for lysis of prokaryotic cells. Purification of nucleic acids, particularly DNA, follows by removal of cellular debris and the lysis agents.

For example, extraction of RNA is often done using a guanidine-isothiocyanate phenol:chloroform extraction method in which protein and DNA are partitioned into the organic layer, while RNA is partitioned into the aqueous layer. A subsequent precipitation of the RNA with an alcohol, such as isopropanol, results in purified RNA. In some variations of this protocol, 2-mercaptoethanol is included in the lysis buffer. Cell lysis and DNA isolation also often relies on phenol:chloroform extraction. However, some methods avoid the use of organics, and rely on preferential precipitation of proteins by a salt, such as with sodium chloride, followed by precipitation of the nucleic acid with alcohol. Other methods rely on solid resins, such as silica-based resins, to bind proteins and separate them from nucleic acids, particularly DNA.

Cells are commonly lysed with N-lauryl sarcosyl detergent and guanidine thiocyanate or lithium chloride (both chaotrophic salts). Nucleic acids are then isolated out of this lysate solution by passing the lysate over a glass fiber filter or mixing it with charged polymeric beads, either of which will bind the nucleic acids. Subsequent washing and elution of the nucleic acids is required.

Where amplification of nucleic acids liberated from the cells is desired, the lysis compositions typically contain substances that inhibit nucleic acid degrading enzymes, such as DNases and RNases, or the composition comprising the nucleic acids are treated with heat to inactivate the nucleic acid degrading enzymes. Among the most common of such substances are diethyl pyrocarbonate (DEPC), phenol, proteases, ionic detergents, and antibody cocktails. Heating to a temperature sufficient to inactivate or denature proteins is a viable step for minimizing nucleic acid degradation. However, this step is often problematic where ribonucleic acids are of interest.

Prior art methods for retrieving nucleic acids from nematodes include physical disruption by cutting (Waeyenberge, et al. 2000. Nematology 2: 135-142), bead beating (Donn et al. 2008. Applied Soil Ecology 38: 20 - 26), rupture with a pestle (Orui. 1996. Applied Entomology and Zoology 31:505-514) and other very laborious and time consuming methods. The chemical / enzymatical nematode lysis protocol as described by Holterman et al. (2006) (Molecular Biology and Evolution 23(9):1792-1800) is faster and far less laborious than the methods described above, however it was designed and will only work for single or very few nematodes (up to 50). For practical purposes such as the analysis of soil or sediment samples - samples typically containing 100 - 10,000 nematodes this method is inappropriate as it will not result in the successful amplification of any DNA fragment.

The present invention now provides a rapid and simple lysis buffer, which allows for lysis of 1 - 10,000 nematodes, preferably terrestrial or freshwater nematodes, isolation of nucleic acids of it and sensitive, quantitative amplification by PCR, without physical and / or mechanical disruption and without intermediate purification steps.

The term "sensitive" refers to the fact that the lysed material using a lysis buffer according to the invention can be directly used in a subsequent amplification step. There is no need to dilute the lysed material prior to amplification in order to reduce, for example, the detergent concentration. For example, if only one or a few nematodes are present in a sample, further dilution of the lysed material might yield false negative results in the subsequent amplification step.

The term "quantitative amplification" is known to the skilled person and refers to the amplification and quantification of a target DNA molecule. Quantitative amplification allows quantification of starting amounts of a DNA or RNA template.

The term "terrestrial and freshwater nematodes" refers to free-living and plant parasitic nematodes, as opposed to animal parasitic nematodes.

### Aqueous buffer

The aqueous buffer used in aspects of the present invention may be any buffer that supports the activity of the endopeptidase, and that conserves the nucleic acids liberated from the nematode cells. Hence, high pH buffers are preferably avoided as this could result in chemical hydrolysis of the released nucleic acids. In the case of proteinase K, which has a wide pH optimum of between pH 7.5-12, a suitable pH for the buffer is 8.0. Preferably the buffer is such that the pH of the composition is between 5.0 and 9.0, preferably between 6.0 and 8.5, more preferably between 7.0 and 8.0.

Suitable buffering agents include, but are not limited to tris[hydroxymethyl]-4-amino-butanesulfonic acid (TABS), 3-{[tris(hydroxymethyl)methyl]-amino}propanesulfonic acid (TAPS), N,N-bis(2-hydroxyethyl)glycine (Bicine), tris(hydroxymethyl)methylamine (Tris), N-tris(hydroxymethyl)methylglycine (Tricine), 4-2-hydroxyethyl-1-piperazineethanesulfonic acid (HEPES), 2-{[tris-(hydroxymethyl)methyl]amino}ethanesulfonic acid (TES), 3-(N-morpholino)-propanesulfonic acid (MOPS), piperazine-N,N'-bis(2-ethanesulfonic acid) (PIPES), dimethylarsinic acid (Cacodylate), 2-(N-morpholino)ethanesulfonic acid (MES), sodium or potassium citrate, citric acid, boric acid, sodium bicarbonate, sodium borate, and various mixed phosphate buffers including combinations of Na₂HPO₄, NaH₂PO₄ and KH₂PO₄. Of these, Tris-HCL is preferred.

Generally, buffers may be used in amounts ranging from about 0.05 to 5% with the preferred concentration being 0.5 to 2.0%. An amount of 1.5 wt% of Tris-HCl is highly preferred.

The aqueous buffer may further comprise one or more electrolytes or salts, such as a sodium salt, a potassium salt, a magnesium salt, a manganese salt, a zinc salt, a cobalt salt, or a combination of two or more of these salts. Specific exemplary salts include sodium chloride, magnesium chloride, manganese chloride, and potassium chloride. The salts may be added in any suitable amount and for any reason such as NaCl. Suitable concentrations of salt are between 0.1 and 1 wt%. An amount of 0.6 wt% of NaCl provides very good results.

Preferably, an aqueous buffer comprises 100 mM NaCl and 100 mM Tris-HCl (pH 8).

The composition will typically contain a solvent, such as water, an organic solvent, or both. Although it is preferred that the solvent used be as pure as possible or practicable, solvents of any purity may be used. Thus, where water is included in the composition, it may be distilled water, double-distilled water, de-ionized water, sterilized water, or any combination thereof. The solvent, be it water or any other solvent or combination of water and any other solvent, may be treated before use to reduce or eliminate one or more chemical or biochemical activities, such as, but not limited to nuclease (e.g., RNase, DNase) activities. Likewise, the composition may be treated with sterilization techniques or with chemicals or biologicals, etc. to sterilize the composition or to reduce or eliminate one or more undesirable chemical or biochemical activities (e.g., RNase, DNase, etc.).

### Reducing agent

The reducing agent in a composition of the invention is suitably selected from the group consisting of methanethiol, ethanethiol, 1-propanethiol, 2-propanethiol, butanethiol, tetrabutyl mercaptan, pentanethiol, 2-mercaptoethanol, tris (2-carboxyethyl)phosphine (TCEP), dithiothreitol (DTT), cysteamine, glutathione, cysteine and redox mixtures thereof.

In preferred embodiments, the reducing agent is 2-mercaptoethanol.

Suitable amounts of the reducing agent include final concentrations in the composition of the invention ranging from 0.001-0.9 wt %, more preferably 0.1-0.8 wt%, still more preferably 0.25-0.75 wt. %, still more preferably about 0.5 wt.%.

### Endopeptidase

As endopeptidase such proteases as trypsin, chymotrypsin, pepsin, papain, elastase, proteinase K and/or protease type XXI (pronase) are described herein. In embodiments of the present invention said endopeptidase is proteinase K. The amounts of endopeptidase can be selected such as to provide sufficient proteolytic activity in the composition of the invention, which may depend on the endopeptidase used as well as the pH of the aqueous buffer solution, and the speed of the lysis needed. Proteinase K is preferably used at a concentration ranging from 0.01-0.1 wt%, more preferably 0.02-0.06 wt%. Highly preferred is a concentration of 0.04 wt% of proteinase K in a composition of the present invention.

### Chelating agent

The chelating agent is an optional compound which may be beneficial in circumstances wherein the sample comprises large amounts of organic matter. Such organic matter, notably humic compounds, is believed to interfere with the nucleic acid amplification reaction. Suitably, the chelating agent is selected from the group consisting of phosphonate chelating agents, amino carboxylate chelating agents, carboxylate chelating agents, polyfunctionally-substituted aromatic chelating agents and mixtures thereof. Preferably, the chelating agent is selected from the group consisting of hydroxyethanediphosphonic acid (HEDP), nitrilotrimethylenephosphonic acid (NTMP), ethylenediaminetetramethylenephosphonic acid (EDTMP), diethylenetriaminepentamethylenephosphonic acid (DTPMP), ethylenediaminedisuccinic acid (EDDS), ethylenediaminetetraacetic acid (EDTA), cyclohexanediaminetetraacetic acid (CDTA), propylenediaminetetracetic acid (PDTA), diethylenetriaminepentaacetic acid (DTPA), methylglycinediacetic acid (MGDA), hydroxyethylenediaminetriacetic acid (HEDTA), nitrilotriacetic acid (NTA), ethylenediaminetetrapropionic acid (EDTP), triethylenetetraaminehexaacetic acid (TTHA), hexametaphosphate (HMP), potassium pyrophosphate, sodium tripolyphosphate, ethanoldiglycine, malonic acid, salicyic acid, glycine, aspartic acid, glutamic acid, dipicolonic acid, and mixtures thereof, preferably from the group consisting of EDTA, CDTA, HEDTA, DTPA, and hexametaphosphate (HMP). Preferably the chelating agent is EDTA.

The amount of chelating agent may vary depending on the application, but in general may comprise from 0.0 - 0.5 wt%, preferably from 0.001-0.5 wt%. Preferred concentrations range from 0.01-0.3 wt%, more preferred from 0.07- 0.2 wt%, more preferred from 0.01-0.1 wt%. Highly preferred is an amount of between 0.03 and 0.15 wt%, such as 0.07 wt% and 0.15 wt%. An amount of 0.03 wt% of EDTA has shown to give very good results.

### Anionic surfactant

A composition of the present invention further comprises an anionic surfactant Preferably, the anionic surfactant is selected from the group consisting of sodium dodecyl sulphate (SDS or NaDS), sodium decyl sulfate (SDeS), sodium pentanesulfonate (SPS), sodium octanesulfonate (SOS), and sodium N-lauroyl-N-methyltaurate (LMT), linear alkyl sulfonate, linear alkylbenzene sulfonate, sodium laureth sulphate (SLES) and mixtures thereof, preferably SDS.

The amount of anionic surfactant in a composition of the invention is suitably from 0.0 - 0.5 wt%, preferably from 0.001-0.1 wt%. Preferred amounts are below 0.1 wt%, such as 0.05 wt% or preferably 0.025 wt% or 0.013 wt% of the anionic surfactant.

The weight or volume ratio of a reducing agent in relation to an anionic surfactant is preferably higher than 5:1, more preferably between 10:1 and 50:1, still more preferably about 20:1. The molar ratio of reducing agent over anionic surfactant is preferably higher than 10, more preferably between 50 and 100, still more preferably around 70.

In a most preferred embodiment, the composition comprises 0.025 wt% SDS (0.9 mM); 0.5% (v/v) 2-mercaptoethanol (70 mM) and 400 mg/L proteinase K in an aqueous buffer, further comprising 1 mM EDTA (0.03 wt%).

A method for amplifying nucleic acids from nematodes or pant cells according to the present invention comprises the step of providing a sample of nematodes or plant cells. The nematode can be any nematode species cell. In exemplary embodiments, the nematode is a member of the order Enoplida, Triplonchida, Dorylaimida, Mononchida, Isolaimida, Mermithida, Trichinellida, Chromadorida, Desmodorida, Desmoscolecida, Monhysterida, Araeolaimida, Plectida, Rhabditida (including e.g. the infraorders Panagrolaimomorpha, Cephalobomorpha and Tylenchomorpha) as defined by De Ley, Decreamer and Eyualem-Abebe *in* Freshwater Nematodes - Ecology and taxonomy, edited by Eyualem-Abebe, Traunspurger and Andrássy (CAB International 2006).

Nematodes may be extracted from soil or plant material by any method available in the art. Among nematode extraction techniques active and passive methods are distinguished. Active techniques depend on the behavior of soil animals, whereas passive techniques depend on their specific gravity or sedimentation rate. Moreover, distinct experimental set ups are used for the extraction of nematodes from soil and sediments, and from plant material (roots, stems or leaves). The Oostenbrink elutriator (passive technique) is suitable for extraction of vermiform and vital nematodes from soil, sediment, manure and other substrates. The elutriator exploits differences in density and shape between nematodes and soil particles. Within the Oostenbrink elutriator, an upward water flow causes relative low density particles (such as nematodes) to stay afloat whereas soil particles will settle. Via a side-exhaust the suspension will be tapped and subsequently sieved to remove small soil particles.

Another passive technique uses an extracting solution, usually sugar or a salt in water, with a specific gravity sufficient to suspend the nematodes but which allows soil particles to settle out. The most widely used passive technique for nematode extraction is centrifugation (Caveness and Jensen, 1955. Proc. Helminthol. Soc. Wash., 22: 87-89). The sample is then suspended in water and centrifuged to precipitate nematodes and soil particles and remove organic debris in the supernatant. The sample is then re-suspended in a high density sugar solution (specific gravity = 1.10-1.18) to precipitate soil particles and suspend nematodes in the supernatant for easy collection.

Alternatively, a Baermann funnel could be used (active technique). In this method, substrate - soil, sediment, manure - is wetted and placed on a porous filter (tissue paper, cloth) supported by a funnel. Nematodes move out of the substrate toward the water, and sink to the bottom of the (water-filled) funnel stem due to gravity forces. This method is only suitable for mobile nematodes.

All techniques will result in a relatively clean and fairly concentrated nematode suspension. For further concentration, aliquots of 2 ml are transferred to microcentrifuge tubes, centrifuged at 15,000 x g for 10 min. After removal of most of the supernatant, the nematode pellet is left in approximately 300 µl water and stored at -20°C.

It is highly preferred that for nematode samples, use is made of (siliconized) centrifuge tubes when concentrating the nematode sample, since nematodes stick vigorously to normal plastics by electrostatic interaction.

The plant cell can be any type of plant cell, including cells from leaves, non-lignified stems, flowers and non-lignified root material. This approach was tested and shown to work for a number of solanaceous plant species including tomato, potato and tobacco. Preliminary experiments with leaf fragments from randomly selected monocotyledonous and dicotyledonous weeds indicate that this lysis protocol can be used for DNA extraction from non-lignified plant material in general.

The number of nematodes or plant cells included in the composition (before lysis), and thus the volume of nematodes, can vary depending on nematodes type. Numerous nematodes or plant cell types from various origins, which can be isolated directly from soil or crops, grown in culture media, collected on filter paper, dried, etc., can be included in the compositions of the inventions. Thus, the nematodes or plant cells can be samples of, or originate from, environmental samples, including, but not limited to soil, sludge, vegetation, roots, water and air. Typically, the number of nematodes or plant cells present in a composition ranges from about 1 nematode to about 100,000 nematodes or plant cells.

A method for amplifying nucleic acids from nematodes or plant cells according to the present invention comprises the step of lysing the nematodes or plant cells using the composition of the present invention to provide a nematode- or plant cell-lysate. The method comprises contacting at least one nematode or plant cell with a composition of the invention for a sufficient amount of time to cause the nematode or plant cell to lyse. The contact between the nematode or plant cell and the composition is preferably maintained for an effective duration, that is, until lysis of the nematodes or plant cells has occurred quantitatively. Due to the chemical nature of the lysis reaction, it is envisioned that the time can be quite short, on the order of 1 second or less. However, the time is not so limited. Indeed, because it has been found that the compositions of the invention can provide long-term storage of nucleic acids, the time for lysis of nematodes or plant cells can be relatively long. Suitable times can range from 1 second or less to minutes, hours, or days. Exemplary times for exposure of the nematodes or plant cells to the lysis conditions (before nucleic acid extraction) include, but are not limited to, 5 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, overnight and 1 day. Hence, the invention provides in one aspect a method of lysing nematodes or plant cells using a composition of the invention.

A method for amplifying nucleic acids from nematodes or plant cells according to the present invention further comprises the step of isolating the nucleic acids from said nematode- or plant cell-lysate. Although there are many suitable methods available, such as using chaotropic agents, the use of methods that require no further purification of the nucleic acids is preferred.

In addition, the compositions of the invention have surprisingly been found to be suitable for amplification of nucleic acids. It has been found that the specific concentrations of components used allow for the lysis of the nematodes but do not interfere with the specificity or sensitivity of amplification of nucleic acids by PCR techniques. Thus, in embodiments, compositions of the invention may comprise at least one reagent that is used in PCR. For example, in embodiments, the compositions may comprise at least one primer for amplification of a target nucleic acid. The composition may also comprise a probe, such as a TaqMan probe, a molecular beacon, or a scorpion probe, in the composition. In embodiments, the compositions may comprise at least one polymerase, including, but not limited to a thermostable polymerase and a reverse transcriptase. In embodiments, the compositions may comprise one or more nucleotides or nucleotide analogs, which can be incorporated into a growing nucleic acid chain being synthesized by a polymerase. In embodiments, a detecting agent for amplified nucleic acids is present in the composition. Such detecting agents are well known, and include, without limitation, SYBR green, radiolabeled nucleotides, a TaqMan probe, a molecular beacon, a scorpion probe, or any other suitable fluorescent or chemiluminescent compound or combination of compounds. In essence, because the compositions of the invention are suitable for amplification of nucleic acids, any one or more reagent that is known to be useful in an amplification reaction, such as one of the many PCR techniques, can be present in a composition of the invention.

Also described herein are kits. In general, the kits contain a composition of the invention. The kits can further comprise one or more substances, materials, reagents, etc. that can be used for lysis of cells, storage of nucleic acids or cell lysates, or manipulation or analysis of nucleic acids, such as amplification of nucleic acids. In embodiments, some or all of the materials, reagents, etc. necessary to lyse cells, amplify nucleic acids, and/or purify nucleic acids are included in the kit.

The kits can comprise the components in a single package or in more than one package within the same kit. Where more than one package is included within a kit, each package can independently contain a single component or multiple components, in any suitable combination. As used herein, a combination of two or more packages or containers in a single kit is referred to as "in packaged combination". The kits and containers within the kits can be fabricated with any known material. For example, the kits themselves can be made of a plastic material or cardboard. The containers that hold the components can be, for example, a plastic material or glass. Different containers within one kit can be made of different materials. In embodiments, the kit can contain another kit within it. For example, the kit as described herein can comprise a kit for purifying nucleic acids.

For example, a kit may contain one or more containers holding the lysis composition of the invention in pre-mixed form, in concentrated form e.g. as a concentrated stock solution, for example, as a 50x solution, or in a form wherein individual components such as 2-mercaptoethanol and proteinase K are provided separate from the remaining components of the composition, in the same or a separate container, optionally in combination with at least one reagent for amplification of a target nucleic acid. Thus, it can comprise at least one primer, such as two primers, for amplification of a target nucleic acid. It also may include at least one other primer for amplification of a target nucleic acid, which can be, but is not necessarily, the same nucleic acid (and even the same sequence within the same nucleic acid) that is the target for one or more other primer(s) in the kit. In some embodiments, the kits comprise two or more primers for amplifying one or more unique genomic sequences.

A kit as described herein may be a kit for analysis of nucleic acids. Thus, for example, it can be a kit for detection of RNA using a one-step QRT-PCR, such as the Brilliant® QRT-PCR kit from Stratagene or the Full Velocity® QRT-PCR kit from Stratagene, further comprising in at least one container the lysis composition of the invention. Such kits can comprise, in packaged combination, at least one reverse transcriptase, at least one DNA polymerase, such as *Taq* DNA polymerase, *Pfu* DNA polymerase, *Pfx* DNA polymerase, *Tli* DNA polymerase, *Tfl* DNA polymerase, and klenow, an RNase inhibitor, nucleotides (e.g., any or all of the four common deoxynucleotides), primers, probes, or labels (such as, for example, SYBR green), or any combination of two or more of these. Alternatively, it can be a kit that can be used for detection of RNA using a two-step QRT-PCR, such as the StrataScript® First Strand cDNA synthesis kit (which can be used in conjunction with other kits and protocols, such as those mentioned above). Alternatively, the kit can be one that can be used for detection of DNA using a PCR technique, such as QPCR. In addition, the kit can be one that is used for detection of short interfering RNA (siRNA). In embodiments, the kits may comprise transfection or transformation reagents.

The kit as described herein can comprise one or more components useful for amplifying target sequences. In embodiments, some or all of the reagents and supplies necessary for performing PCR are included in the kit. In exemplary embodiments, some or all reagents and supplies for performing QPCR are included in the kit. In other exemplary embodiments, some or all reagents and supplies for performing RT-PCR are included in the kit.

The kit can comprise at least one dye for detecting nucleic acids, including, but not limited to, dsDNA. In embodiments, the kit comprises a sequence-non-specific dye that detects dsDNA, such as SYBR® Green dye (Molecular Probes, Eugene, Oreg.). The kit can also comprise one or more components useful for purifying nucleic acids. In embodiments, these components are particularly suited for purifying target nucleic acids from eukaryotic cell cultures. The components can be, among other things, reagents and supplies that can be used to purify nucleic acids. Non-limiting examples of such reagents and supplies include, but are not limited to, a DNA binding solution, a wash buffer, and containers, such as microfuge tubes, for collection of binding solutions, wash buffers, and purified nucleic acids. The components can also contain a resin, gel, or other substance that is useful for purifying nucleic acids.

### EXAMPLES

### Example 1. Isolation of DNA from nematodes

Methods and compositions for DNA extraction from nematodes are described herein below. The method essentially comprises the following steps:
1) Sample preparation: extraction of nematodes from substrates (examples of the technical procedures are given above.
2) Concentration of the nematode sample: usually this will be done by centrifugation followed the removal of the supernatant.

### 2) Lysis

A. Proteinase K (20 mg/mL)
   Proteinase K (Merck, 1.24568.0500) is dissolved in ultrapure water to 20 mg/mL. 400 µL ±20 µL aliquots are prepared to provide stock solutions which are kept in the freezer.
B. 4x Nematode buffer (400 mM NaCl / 400 mM Tris-HCl, pH 8 / 4 mM EDTA, pH 8/0.1 wt% SDS) is prepared as stock solution for the lysis composition using ultrapure water. The solution is heat sterilized before use and is stored at room temperature.
C. Lysis composition (100 mM NaCl / 100 mM Tris-HCl, pH 8/ 1 mM EDTA / 0.025 wt% SDS / 0.5% 2-mercaptoethanol (v/v) en 400 µg/mL proteinase K)
   The lysis composition is prepared from:
   - 50 µL (±5µL) of 4x Nematoden Lysisbuffer (see B.)
   - 4 µL (±0.4 µL) of proteinase K (stock solution = 20 mg/mL; see A.)
   - 1 µL (±0.1 µL) of 2-mercaptoethanol (PlusOne; 17-1317-01)
   - 150 µL (±7.5 µL) of water
- Proteinase K and 2-mercaptoethanol are added just prior to use.

### Procedure for lysis:

1. Heat an incubator to 65°C ± 3°C.
2. Prepare the lysis composition as indicated under C. above.
3. Add to each nematode sample 205 µL ± 10 µL of lysis composition.
4. Close the sample tubes.
5. Vortex the tubes to re-suspend the nematode pellet in the lysis composition.
6. Incubate and stir the tubes with the nematode-lysis composition for a minimum of 30 min and preferably no more than 3 hrs. in the pre-heated incubator (a Thermomixer (Eppendorf) is an example of an adequate device for nematode lysis).
7. After incubation transfer the lysate to a new tube.
8. Centrifugate for 1 minute at 9000 rpm ± 900 rpm in a microcentrifuge
9. For amplification the supernatant thus obtained contains the nucleic acids, and this template containing solution may optionally be diluted for up to 100 x or more, or purified further using for instance a glass fiber-based DNA purification method. An example of such a protocol is described in Ivanova NV, de Waard J, Hebert PDN (2006) ("An inexpensive, automation friendly protocol for recovering high-quality DNA") Molecular Ecology Notes, 6, 998 - 1002. The nature of the substrate determines whether or not a simple 100 x or more dilution will be sufficient to neutralize the effect of potentially PCR inhibiting components. Especially in case of high organic matter contents or in case of peaty soils, a nucleic acid purification step will be required.

Results of the lysis composition on nematodes are provided in Figure 1. The effect on the addition of SDS on the lysis efficiency of nematodes is shown in Figure 2.

### Example 2. Isolation of DNA from plant tissue

As illustrated by screening of *Nicotiana tabacum* for stable transformants.

### PCR lysis composition (2 times concentrated):

### Composition:

- 200mM NaCL
- 200mM Tris-HCl pH=8
- 2 mM EDTA
- 0.05 wt% SDS

### Added just before use:

- 1% 2-mercaptoethanol
- 800 µg/ml Proteinase K

An amount of ~ 5 mg plant tissue was used for analysis and was treated as described above in Example 1. The method requires no homogenization, and the nucleic acid template was PCR-ready within 3 hours (almost irrespective of the number of samples treated). Lysis could be performed in 96 well format, making further processing easier using a multi-channel pipette. Using the lysis composition ~ 400 transformants were screened within 3 days.

Results of the amplification reaction with the lysis composition are provided in Figure 3.

### Example 3. Isolation of RNA from nematodes

As illustrated by RNA isolation from the potato cyst nematode *Globodera rostochiensis*. With respect to homogenization *G. rostochiensis* is one of the most robust plant parasitic nematode species.

PCR lysis composition was used as described in Examples 1 and 2 to lyse the nematodes. RNase-free proteinase K was used. The procedure as described in Example 1 was used and was followed by RNA extraction with RNeasy Mini Kit.

The RNA recovery was very good and the RNA obtained was of good quality (no contamination at 230 nm). There was no interference with one-step RT-PCR analysis of gene expression. Using this method it was confirmed that dsRNA treated *Globodera rostochiensis* show decreased expression of VAP-1. The results are presented in Figure 4.

### Example 4. Isolation of DNA from nematodes using different SDS concentrations

To test the influence of the SDS concentration in the lysis buffer on lysis and subsequent amplification, the following analysis was performed.

*Globodera rostochiensis* R9-11A were hatched by soaking for 6 days in water that was purified by ion exchange (MQ) followed by soaking in PRD (Potato root diffusate). G. *rostochiensis* was collected in 10ml MQ and washed 3X using MQ. Nematodes were allowed to settle for 1hr. Subsequently, the upper phase was removed using a vacuum pomp and sterile MQ was added. This step was repeated 3X. Finally *G. rostochiensis* was transferred into a final volume of 60ml sterile MQ followed by a classical counting. While bubbling, 28 equal nematode portions of 1ml were extracted from the suspension using a cut pipet tip to get a bigger opening. After spinning (2 min 12.000rpm), each portion was taken up in a volume of 250 µl MQ. Subsequently 250 µl 2X lysis buffer was added according to Table 1 and incubated 1hr at 65°C at 750rpm (Thermomixer).

**Table 1: Composition of PCR lysis buffers (2 times concentrated):**

| Component | LB1 | LB2 | LB3 | LB4 | LB5 | LB6 | LB7 |
|---|---|---|---|---|---|---|---|
| 200 mM NaCl | + | + | + | + | + | + | + |
| 200 mM Tris-HCl | + | + | + | + | + | + | + |
| SDS (w/v %) | 0,00 | 0,01 | 0,025 | 0,5 | 0,1 | 0,5 | 1 |
| 1% 2-mercaptoethanol (w/v) | + | + | + | + | + | + | + |
| 800 µl/ml Proteinase K* | + | + | + | + | + | + | + |
| Internal control | + | + | + | + | + | + | + |

| | | | | | | | |
|---|---|---|---|---|---|---|---|
| *: 2-Mercaptoethanol and Proteinase K were added just before use. | | | | | | | |

Lysis was followed by an inactivation step (10 min 99°C). The resulting crude extracts were stored at -20°C for Q-PCR. Four replicate samples (4X1ml nematode suspension) and 1 blank were processed for each lysis condition. Blank sample consists of 250 µl MQ and 250 µl of 2X LB.

PCR mix was assembled according to Table 2.

**Table 2**

| Component | Amount |
|---|---|
| MQ | 8 µl |
| iQ-sybergreen (Absolute Sybr Green Fluorescein Cat.CM-225; Westburg) | 12,5 µl |
| Primer 1 (5µM stock) | 1 µl |
| Primer 2 (5µM stock) | 1 µl |
| Template (undiluted) | 2,5 µl |
| Total | 25 µl |

18S rDNA Q-PCR primers that were used are:
Primer 1 (Primer No: 8) 5' -TTTACGGTYAGAACTAGGG (Nematode specific)
Primer 1 (Primer No: 103) 5' -GAGGGCAAGTCTGGTG (Eukaryote universal)

Control primers that were used are:
Control primer 1 (Primer No: 1980): 5'- CATAGTAGGCCTAAAAGCAGCC (Eukaryote specific)
Control primer 1 (Primer No: 981) 5'- TGGGGCTAGGAGTTAATCATTTG (Eukaryote specific)

Q-PCR settings were:
94°C 15 min (1*)
94°C 30 sec; 63°C 60 sec; 72°C 30 sec (60*)
75°-90°C 10 sec 31 +0,5°C (Melt curve)

The results are presented in Figures 5 and 6:

### Conclusion

When PCR is performed directly after lysis without any further purification, an SDS concentration window from 0 - 0.05 wt% (in 1*lysis buffer), results in good amplication of the DNA. A more preferred SDS concentration is between 0.013 wt% to 0.05 wt% in lysis buffer, resulting in 0.0013 wt% to 0.005 wt% in PCR reaction tube. Higher SDS concentrations result in inhibition of the PCR reaction. Lower SDS concentrations result in less efficient lysis, as indicated by the higher Ct values that were obtained (see Figure 6).

### Example 5. Isolation of DNA from nematodes using different EDTA concentrations

To test the influence of the EDTA concentration in the lysis buffer on lysis and subsequent amplification, the following analysis was performed.

*Globodera rostochiensis* R9-11A were hatched by soaking for 6 days in water that was purified by ion exchange (MQ) followed by soaking in PRD (Potato root diffusate). G. *rostochiensis* was collected in 10ml MQ and washed 3X using MQ. Nematodes were allowed to settle for 1hr. Subsequently, the upper phase was removed using a vacuum pomp and sterile MQ was added. This step was repeated 3X. Finally *G. rostochiensis* was transferred into a final volume of 60ml sterile MQ followed by a classical counting. While bubbling, 28 equal nematode portions of 1ml were extracted from the suspension using a cut pipet tip to get a bigger opening. After spinning (2 minutes 12.000rpm), each portion was taken up in a volume of 250 µl MQ. Subsequently 250 µl 2X lysis buffer was added according to Table 1 and incubated 1hour at 65°C at 750rpm (Thermo mixer).

**Table 3. Composition of PCR lysis buffers (2 times concentrated):**

| Component | LB1 | LB2 | LB3 | LB4 | LB5 | LB6 | LB7 |
|---|---|---|---|---|---|---|---|
| 200 mM NaCl | + | + | + | + | + | + | + |
| 200 mM Tris-HCl | + | + | + | + | + | + | + |
| SDS (0,05%) (w/v) | + | + | + | + | + | + | + |
| 1% 2-mercaptoethanol (w/v) | + | + | + | + | + | + | + |
| 800 µg/ml Proteinase K* | + | + | + | + | + | + | + |
| EDTA in mM (% (w/v)) | 0 | 2 | 5 | 10 | 20 | 25 | 50 |
| | (0) | (0,06) | (0,15) | (0,29) | (0,58) | (0,73) | (1,46) |

| | | | | | | | |
|---|---|---|---|---|---|---|---|
| *: 2-Mercaptoethanol and Proteinase K were added just before use. | | | | | | | |

Lysis was followed by an inactivation step (10 min 99°C). The resulting crude extracts were stored at -20°C for Q-PCR. Four replicate samples (4*1 ml nematode suspension) and 1 blank were processed for each lysis condition. Blank sample consists of 250 µl MQ and 250 µl of 2* LB.

PCR mix was assembled according to Table 2, using the 18S rDNA Q-PCR primers as indicated in Example 4.

The results are presented in Figures 7 and 8:

### Conclusion

Good results were obtained using EDTA concentrations between 0 and 1 mM in the final PCR reaction mixture, corresponding to between 0 and 10 mM (between 0 and 0.29 wt%) in the 1*lysis buffer. More preferred EDTA concentrations are between 2.5 and 10 mM (between 0.07 and 0.29 wt%) in the 1*lysis buffer, resulting in EDTA concentrations between 0.25 and 1 mM in the final PCR reaction mixture. Most preferred EDTA concentrations are between 2.5 and 5 mM (between 0.07 and 0.14 wt%) in the 1*lysis buffer, resulting in EDTA concentrations between 0.25 and 0.5 mM in the final PCR reaction mixture. Higher EDTA concentrations result in inhibition of the PCR reaction. Lower EDTA concentrations result in less efficient lysis, as indicated by the higher Ct values that were obtained (see Figure 8).

### Example 6. The effect of SDS concentration on lysis efficiency using several incubation times and G. rostochiensis.

*Globodera rostochiensis* R9-11A was hatched and aliquoted as described in Examples 4 and 5. The 2*concentrated lysis buffers that were used are indicated in Table 4.

Samples of lysing nematodes were taken at different time points using the following strategy: After incubation (for example 5 minutes) lysates were spinned (30 sec 12.000rpm) to pellet undissolved debris. Next 50 µl of the supernatant was pipetted and transferred into a new 1,5ml tube followed by inactivation at 99°C and diluted 20 times by adding 950 µl MQ. The 950 µl lysate that was left over was placed back immediately for lysis. This was repeated for all time points up to 120minutes. Three replicate samples (3* 1ml nematode suspension) and 1 blank were processed for each incubation time and lysis method. Blank sample: 500 µl 2*LB+500 µl MQ.

**Table 4: Composition of PCR lysis buffers (2 times concentrated):**

| Component | LB1 | LB2 | LB3 |
|---|---|---|---|
| 200 mM NaCl | + | + | + |
| 200 mM Tris-HCl | + | + | + |
| SDS (w/v %) | 0,00 | 0,05 | 1 |
| 1% 2-mercaptoethanol (w/v) | + | + | + |
| 800 µg/ml Proteinase K* | + | + | + |
| Internal control | + | + | + |

| | | | |
|---|---|---|---|
| *: 2-Mercaptoethanol and Proteinase K were added just before use. | | | |

PCR mix was assembled according to Table 2, using the 18S rDNA Q-PCR primers as indicated in Example 4.

The results are presented in Figure 9:

### Conclusion:

The use of low concentrations of SDS results in efficient and consistent lysis of the samples within 30 minutes of incubation. High amount of SDS (1 wt%) result in a faster lysis within 5-10 minutes, but these samples are not suitable for direct PCR analysis (see Example 4; please note that the present samples were diluted 50* before PCR analyses). The use of undiluted samples without further purification in a subsequent amplification reaction will strongly enhance the sensitivity of the detection of nematodes..

## Claims

1. A composition for lysis of nematodes , which composition, upon lysis of the nematodes therein, provides a nucleic acid template solution for use in a subsequent amplification reaction without further purification, said composition consisting of an aqueous buffer containing:
- 0.01-0.5 wt% of a reducing agent,
- 0.01-0.5 wt% of proteinase K,
- 0.001-0.1 wt% of an anionic surfactant, and
- 0-0.5 wt% of a chelating agent.

2. The composition of claim 1, wherein said composition consists of an aqueous buffer containing:
- 0.5 wt% of a reducing agent,
- 0.04 wt% of proteinase K,
- 0.025 wt% of an anionic surfactant, and
- 0-0.15 wt% of a chelating agent.

3. The composition of claim 2, wherein said composition consists of an aqueous buffer containing:
- 0.5% (v/v) of 2-mercaptoethanol,
- 0.04 wt% of proteinase K,
- 0.025 wt% of SDS, and
- optionally further comprising 0.03 - 0.15 wt% of EDTA.

4. The composition of any one of claims claim 1-3, wherein said aqueous buffer further contains one or more electrolytes or salts, preferably between 0.1 and 1 wt% of salts.

5. The composition of any one of claims claim 1-4, wherein a buffer is used in amount ranging from about 0.05 to 5%.

6. The composition of claim 5, wherein said aqueous buffer further contains 0.6 wt% of NaCl and 1.5 wt% of Tris-HCl pH 8.0.

7. A method of lysing a nematode, said method comprising exposing the nematode to a composition according to any one of claims 1-6 for a sufficient amount of time for lysis to occur.

8. A method for isolating nucleic acids from a nematode, said method comprising exposing the nematode to a composition according to any one of claims 1-6 for a sufficient amount of time for lysis to occur in order to provide a nematode lysate and recovering nucleic acids from said lysate.

9. The method of claim 8, wherein said exposure is maintained for between 0.5 and 4 hours at a temperature of between 35 and 70 °C, preferably 2 hours at 65 °C.

10. A method of amplifying a nucleic acid of interest from a nematode, said method comprising the steps of performing a method of any one of claims 8-9 thereby providing a nucleic acid, and subjecting the nucleic acid thus provided to conditions that result in amplification of the nucleic acid of interest.

11. The method of claim 10, wherein subjecting comprises performing a PCR amplification of the nucleic acid of interest.

12. The method of claim 11, further comprising, prior to amplifying, reverse transcribing the RNA in said nucleic acid into cDNA.

13. A method for producing a composition according to any one of claims 1-6, said method comprising mixing the said reducing agent, proteinase K, chelating agent, and anionic surfactant in the indicated amounts in an aqueous buffer to form said composition.

## Patentansprüche

1. Zusammensetzung zur Lyse von Nematoden, wobei die Zusammensetzung, bei Lyse der Nematoden darin, eine Nukleinsäure-Matrizenlösung zur Verwendung in einer anschließenden Amplifikationsreaktion ohne weitere Reinigung bereitstellt, die Zusammensetzung bestehend aus einem wässrigen Puffer, enthaltend:
- 0,01-0,5 Gew.-% eines Reduktionsmittels,
- 0,01-0,5 Gew.-% Proteinase K,
- 0,001-0,1 Gew.-% eines anionischen Tensids und
- 0-0,5 Gew.-% eines Chelatisierungsmittels.

2. Zusammensetzung nach Anspruch 1, wobei die Zusammensetzung aus einem wässrigen Puffer besteht, enthaltend:
- 0,5 % eines Reduktionsmittels,
- 0,04 Gew.-% Proteinase K,
- 0,025 Gew.-% eines anionischen Tensids und
- 0-0,15 Gew.-% eines Chelatisierungsmittels.

3. Zusammensetzung nach Anspruch 2, wobei die Zusammensetzung aus einem wässrigen Puffer besteht, enthaltend:
- 0,5 % (v/v) 2-Mercaptoethanol,
- 0,04 Gew.-% Proteinase K,
- 0,025 Gew.-% SDS und
- optional ferner umfassend 0,03 - 0,15 Gew.-% EDTA.

4. Zusammensetzung nach einem der Ansprüche 1-3, wobei der wässrige Puffer ferner einen oder mehrere Elektrolyte(n) oder Salze enthält, bevorzugt zwischen 0,1 und 1 Gew.-% Salze.

5. Zusammensetzung nach einem der Ansprüche 1-4, wobei ein Puffer in einer Menge im Bereich von etwa 0,05 bis 5 % verwendet wird.

6. Zusammensetzung nach Anspruch 5, wobei der wässrige Puffer ferner 0,6 Gew.-% NaCl und 1,5 Gew.-% Tris-HCl pH 8.0 enthält.

7. Verfahren zum Lysieren eines Nematoden, das Verfahren umfassend Aussetzen des Nematoden einer Zusammensetzung nach einem der Ansprüche 1-6 während eines ausreichenden Zeitraums zum Stattfinden der Lyse.

8. Verfahren zum Isolieren von Nukleinsäuren von einem Nematoden, das Verfahren umfassend Aussetzen des Nematoden einer Zusammensetzung nach einem der Ansprüche 1-6 während eines ausreichenden Zeitraums zum Stattfinden der Lyse, um ein Nematodenlysat bereitzustellen und Nukleinsäuren von dem Lysat zurückzugewinnen.

9. Verfahren nach Anspruch 8, wobei das Aussetzen für zwischen 0,5 und 4 Stunden bei einer Temperatur von zwischen 35 und 70°C, bevorzugt 2 Stunden bei 65°C, aufrechterhalten wird.

10. Verfahren zur Amplifikation einer Nukleinsäure von Interesse von einem Nematoden, das Verfahren umfassend die Schritte von Durchführen eines Verfahrens nach einem der Ansprüche 8-9, dadurch eine Nukleinsäure bereitstellend, und Aussetzen der hierdurch bereitgestellten Nukleinsäure Bedingungen, die in Amplifikation der Nukleinsäure von Interesse resultieren.

11. Verfahren nach Anspruch 10, wobei Aussetzen Durchführung einer PCR-Amplifikation der Nukleinsäure von Interesse umfasst.

12. Verfahren nach Anspruch 11, ferner umfassend, vor der Amplifikation, reverse Transkription der RNA in der Nukleinsäure in cDNA.

13. Verfahren zur Herstellung einer Zusammensetzung nach einem der Ansprüche 1-6, das Verfahren umfassend Mischen des Reduktionsmittels, der Proteinase K, des Chelatisierungsmittels und des anionischen Tensids in den angegebenen Mengen in einem wässrigen Puffer, um die Zusammensetzung zu bilden.

## Revendications

1. Composition pour la lyse de nématodes, laquelle composition, suite à une lyse des nématodes dans celle-ci, permettant d'obtenir une solution de matrice d'acide nucléique pour une utilisation dans une réaction d'amplification ultérieure sans purification supplémentaire, ladite composition consistant en un tampon aqueux contenant :
- 0,01-0,5 % en poids d'un agent réducteur,
- 0,01-0,5 % en poids de protéinase K,
- 0,001-0,1 % en poids d'un tensioactif anionique, et
- 0-0,5 % en poids d'un agent chélateur.

2. Composition selon la revendication 1, où ladite composition consiste en un tampon aqueux contenant :
- 0,5 % en poids d'un agent réducteur,
- 0,04 % en poids de protéinase K,
- 0,025 % en poids d'un tensioactif anionique, et
- 0-0,15 % en poids d'un agent chélateur.

3. Composition selon la revendication 2, où ladite composition consiste en un tampon aqueux contenant :
- 0,5 % (v/v) de 2-mercaptoéthanol,
- 0,04 % en poids de protéinase K,
- 0,025 % en poids de SDS, et
- comprenant en outre facultativement 0,03-0,15 % en poids d'EDTA.

4. Composition selon l'une quelconque des revendications 1 à 3, dans laquelle ledit tampon aqueux contient en outre un ou plusieurs électrolytes ou sels, de préférence entre 0,1 et 1 % en poids de sels.

5. Composition selon l'une quelconque des revendications 1 à 4, dans laquelle un tampon est utilisé en une quantité comprise entre environ 0,05 et 5 %.

6. Composition selon la revendication 5, dans laquelle ledit tampon aqueux contient en outre 0,6 % en poids de NaCl et 1,5 % en poids de Tris-HCl pH 8,0.

7. Procédé de lyse d'un nématode, ledit procédé comprenant l'exposition du nématode à une composition selon l'une quelconque des revendications 1 à 6 pendant une durée suffisante pour qu'une lyse se produise.

8. Procédé pour isoler des acides nucléiques d'un nématode, ledit procédé comprenant l'exposition du nématode à une composition selon l'une quelconque des revendications 1 à 6 pendant une durée suffisante pour qu'une lyse se produise afin d'obtenir un lysat de nématode et de récupérer des acides nucléiques à partir dudit lysat.

9. Procédé selon la revendication 8, dans lequel ladite exposition est maintenue entre 0,5 et 4 heures à une température comprise entre 35 et 70 °C, de préférence 2 heures à 65 °C.

10. Procédé d'amplification d'un acide nucléique d'intérêt d'un nématode, ledit procédé comprenant les étapes consistant à mettre en oeuvre un procédé selon l'une quelconque des revendications 8-9 pour ainsi obtenir un acide nucléique, et à soumettre l'acide nucléique ainsi obtenu à des conditions qui entraînent l'amplification de l'acide nucléique d'intérêt.

11. Procédé selon la revendication 10, dans lequel la soumission comprend la réalisation d'une amplification par PCR de l'acide nucléique d'intérêt.

12. Procédé selon la revendication 11, comprenant en outre, avant l'amplification, la transcription inverse de l'ARN dans ledit acide nucléique en ADNc.

13. Procédé pour produire une composition selon l'une quelconque des revendications 1 à 6, ledit procédé comprenant le mélange desdits agent réducteur, protéinase K, agent chélateur, et tensioactif anionique en les quantités indiquées dans un tampon aqueux pour former ladite composition.
